# EUROPEAN PATENT SPECIFICATION

(11) **EP 2 747 957 B1**
(45) Date of publication and mention of the grant of the patent: **16.12.2015**
(21) Application number: 12823205.5
(22) Date of filing: 18.12.2012
(51) Int. Cl.: B26B 19/00

(54) **HAIR CUTTING DEVICE**
HAARSCHNEIDEVORRICHTUNG
DISPOSITIF DE COUPE DE POILS

(30) Priority: 22.12.2011 US 201161578910 P
(43) Date of publication of application: 02.07.2014
(73) Proprietor: Koninklijke Philips N.V., 5656 AE Eindhoven (NL)
(72) Inventor: DAMKAT, Chris, NL-5656 AE Eindhoven (NL); UZUNBAJAKAVA, Natallia, Eduardauna, NL-5656 AE Eindhoven (NL); CIUHU, Calina, NL-5656 AE Eindhoven (NL); VEENSTRA, Geert, NL-5656 AE Eindhoven (NL); KOOIKER, Harmen, NL-5656 AE Eindhoven (NL)
(74) Representative: Coops, Peter
(86) International application number: PCT/IB2012/057422
(87) International publication number: WO 2013/093772

(56) References cited:
- WO-A1-92/16338
- US-A- 5 182 857
- US-A- 5 993 440

## Description

### FIELD OF THE INVENTION

This invention relates to a hair cutting device comprising cutter unit and a skin stretcher. The cutter unit comprises a skin-contact surface for dragging over a skin surface in a shaving direction, a front surface arranged in front of the skin-contact surface in the shaving direction, and a laser beam exit window arranged in the front surface for allowing a hair cutting laser beam to cut a hair near the skin surface in front of the front surface. The skin stretcher is positioned in front of the cutter unit, according to the shaving direction, and comprises a stretcher surface for dragging over the skin surface together with the skin-contact surface.

### BACKGROUND OF THE INVENTION

Such a hair cutting device is, e.g., known from the international patent application published as WO 2011/010246. Said patent application describes a hair cutting device for cutting hair near skin of a human or animal body part. An optical blade, embodying the functionality of the cutter unit, is dragged over the skin surface, while a reflector in the optical blade directs the hair cutting laser beam to the hair. The optical blade has a tapered end with a curved surface for exerting a local pressure on the skin surface and thereby manipulating the skin in an attempt to improve closeness and minimize skin irritation. Closeness is thereby defined as the length of the remaining stubbles after shaving. Irritation is caused by the hair cutting laser irradiating the skin instead of the hair. WO 2011/010246 further discloses the use of a skin stretcher, installed in front of the optical blade, for stretching the skin and making skin doming more predictable. Skin doming is the piling up of an amount of skin before the front surface of the optical blade when dragging the blade surface over the skin surface in the shaving direction. The aim of this skin manipulation is to ensure that the hair cutting light beam remains parallel to and above the skin surface.

One of the disadvantages of this known hair cutting device is that longer hairs may be trapped between the skin stretcher and the skin while the optical blade passes and will therefore be missed by the hair cutting laser beam. In addition, skin properties like flexibility and smoothness may vary from person to person, within persons on different body sites or positions and perhaps also from day to day, resulting in either reduced closeness or increased irritation.

### OBJECT OF THE INVENTION

It is therefore an object of the invention to provide a hair cutting device which provides improved closeness and reduced irritation.

### SUMMARY OF THE INVENTION

According to a first aspect of the invention, this object is achieved by providing a hair cutting device comprising a cutter unit, a skin stretcher and slit adapting means. The cutter unit comprises a skin-contact surface for dragging over a skin surface in a shaving direction, a front surface arranged in front of the skin-contact surface in the shaving direction, and a laser beam exit window for allowing a hair cutting laser beam to cut a hair near the skin surface in front of the front surface. The skin stretcher is positioned in front of the cutter unit, according to the shaving direction, and comprises a stretcher surface for dragging over the skin surface together with the skin-contact surface, such that a skin dome is formed by the skin surface in a slit between the skin stretcher and the cutter unit. The slit adapting means are provided for adapting at least one dimension of the slit for controlling a shape of the skin dome.

The inventors have realized that the above mentioned problems of the prior art can be avoided by adapting the slit dimensions to obtain an optimum balance between improving closeness and minimizing irritation. For example, when the hairs to be cut are relatively long or the skin surface is relatively smooth and inelastic, larger slits are preferred. Larger slits decrease the chance that a hair is still trapped underneath the stretcher surface when the cutter unit passes. Larger slits also allow more skin to pile up between the skin stretcher and the cutter unit, thereby increasing the skin dome height. So while smooth and inelastic skin surfaces reduce the tendency of skin to pile up, a larger slit may compensate for that effect and increase skin dome height to improve closeness. When the skin properties are such that the skin dome height is larger than usual, a decrease of the slit size reduces the skin dome height and the irritation caused by the hair cutting laser beam hitting the skin.

The inventors have found out that various slit dimension parameters like slit size and exposure are important parameters for manipulating the skin dome shape and height. Slit size is defined as the distance between a rear surface of the skin stretcher and the front surface of the cutter unit. Exposure is defined as the distance between the skin-contact surface and the stretcher surface measured in a direction perpendicular to the shaving direction. By careful tuning the slit size, the exposure or both, the dome shape can be controlled in order to obtain an optimum balance between closeness and irritation.

The slit size and the exposure may both be adapted by either moving the skin stretcher or the cutter unit relative to the hair cutting device. In principle, also moving both parts is an option, but this would require a more complex construction of the device from a mechanical point of view.

Instead of or in addition to adapting the slit size and exposure, also the angle (hereinafter referred to as the cutter angle) between the skin-contact surface and the shaving direction may be adapted. Changing the cutter angle has two effects on the shaving process. A first effect is that the inclined skin-contact surface exerts a different pressure on the skin surface than a flat skin-contact surface would do which may lead to a different dome shape and dome height. A second effect is that, because the light source is rotated together with the cutter unit, the optical base line of the hair cutting laser will also rotate which means that the focus of the hair cutting laser moves away from or towards the skin surface. Both the change of the dome shape and the modification of the optical baseline affect the closeness and the irritation. It is to be noted that the optical baseline may also be modified independent of the cutter unit, e.g. by mechanically rotating or displacing optical elements in the cutter unit.

In an embodiment of the hair cutting device according to the invention, the device may comprise control means for manually setting the appropriate slit dimensions. The user may, e.g., be enabled to select a slit size and/or an exposure from a discrete or continuous range of available settings. Alternatively, the device may offer different settings for short hair and for longer hair. Each setting then corresponds to a different predetermined slit size and/or exposure. Also, different settings for shaving a beard, a head, arms and/or legs may be provided.

Instead of manually setting the slit dimensions, a processor may be provided which is coupled to the slit adapting means for controlling slit dimensions and skin dome height. The processor is preferably also coupled to one or more sensors for measuring parameters that are indicative of the skin dome height. Some examples of such sensors are described below.

The hair cutting device may further comprise a light based hair detector for detecting the hair near the skin surface, a hair cutting laser source for generating the hair cutting laser beam, and a processor coupled to the light based hair detector and to the hair cutting laser source. The processor is arranged to activate the hair cutting laser source in a focal position of the hair cutting laser beam in which the light based hair detector has detected the presence of the hair. Because the device knows the positions of the hairs, the cutting laser can be selectively targeted at the hairs instead of systematically scanning larger areas where hairs may be present. As a result, skin irritation is considerably reduced.

In an embodiment of the hair cutting device according to the invention, the light based hair detector is further adapted to detect a height of the skin dome and the processor is further arranged to control the slit adapting means in dependence of the detected height of the skin dome. The light based hair detector is already equipped to distinguish hairs from skin and may thus also be used for detecting the skin dome height. When the skin dome height is known, it can also be controlled by adapting the slit dimensions. In general, lower skin dome height improves closeness but also increases irritation.

Optionally, the skin stretcher comprises a pressure sensor for determining a pressure exerted on the stretcher surface and wherein the processor is further arranged to control the slit adapting means in dependence of the determined pressure. When a user pushes the hair cutting device into the skin with too much force, the chances of the focal point of the hair cutting laser falling within the skin is considerably increased. To avoid irritation of the skin, the slit dimension may then be adapted in order to reduce skin dome height and thus irritation.

Additionally or alternatively, the skin stretcher comprises a friction sensor for determining a friction between the stretcher surface and the skin surface and wherein the processor is further arranged to control the slit adapting means in dependence of the determined friction. Friction of the skin surface may depend on, e.g., skin type, use of shaving lubricants, hair length and pressure applied by the hair cutting device. More friction leads to a higher skin dome, which can be compensated by appropriate changes to the slit dimensions.

Optionally, also a means for determining a speed of the hair treatment device relative to the skin surface is provided and the processor is further arranged to control the slit adapting means in dependence of the determined speed.

These and other aspects of the invention are apparent from and will be elucidated with reference to the embodiments described hereinafter.

### BRIEF DESCRIPTION OF THE DRAWINGS

In the drawings:
Figure 1 schematically shows a cross section of a hair cutting device according to the invention,
Figure 2 schematically shows a top view cross section of the hair cutting device of figure 1, and
Figure 3 shows the cross section of figure 1, with the cutter unit in a tilted position.

### DETAILED DESCRIPTION OF THE INVENTION

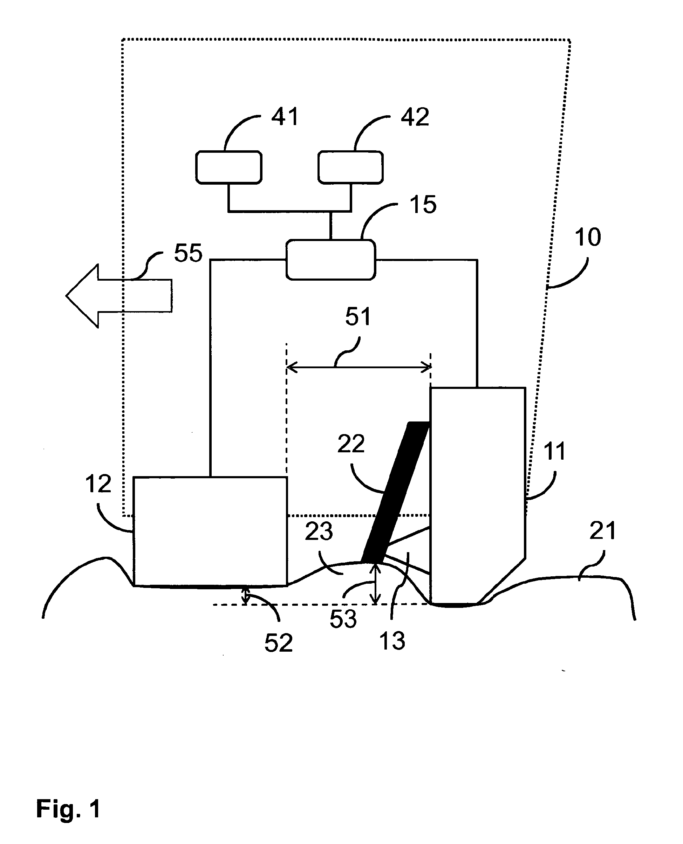

Figure 1 schematically shows a cross section of a hair cutting device 10 according to the invention. The hair cutting device 10 comprises a cutter unit, here in the form of an optical blade 11, optionally with a tapered end as also described in the international patent application published as WO 2011/010246. The optical blade 11 comprises optical elements like mirrors, reflectors and lenses for directing a hair cutting laser beam 13 through an exit window of the optical blade 11 and focusing the hair cutting laser beam 13 in a focal point near the skin surface 21. The optical blade 11 preferably also comprises the light source for the hair cutting laser beam 13. The same or additional optical elements may also be used by a light based hair detector which is used for distinguishing hairs 22 from skin 21. The hair detector and the hair cutting laser may both use the same light source, possibly at a different intensity. Alternatively, separate light sources may be provided for detecting and cutting hairs 22. The light based hair detector and the hair cutting laser are controlled by a processor 15, which is coupled to the light source(s) and the light detector(s) of the light based hair detector. This processor 15 is preferably provided in the body of the hair cutting device 10 and also serves for controlling other parts if the device 10. It is to be noted that the hair cutting device 10 may also function very well without using a hair detector. For example, the hair cutting laser beam 13 may just be focused at a standard height above the skin-contact surface or blade surface and may systematically scan larger areas where hairs 22 may be present.

During shaving, the user moves the hair cutting device 10 over the skin surface 21. The preferred shaving direction 55 is against the grain because this leads to beneficial hair manipulation, e.g. due to additional hair lift, and an improved shaving result. In front of the optical blade 11, a skin stretcher 12 is provided. One of the functions of the skin stretcher 12 is to bring the hairs 22 in a detection area and to achieve hair lift. The detection area is the open space between the optical blade 11 and the skin stretcher 12 where a focused light beam 13 of the hair detector is able to detect the hair 22. The hairs 22 are brought in the detection area by assuring stretching of the skin 21 by the skin stretcher 12 and subsequent contact between the hair 22 and the optical blade 11. Hair lift is achieved due to a shear force, exerted by the skin stretcher 12, and hair rotation around its anchorage point in the skin 21 (due to skin stretching as well as due to hair-optical blade contact).

When dragging the skin stretcher 12 and the optical blade 11 over the skin surface 21, a skin dome 23 is formed in the slit between the stretcher 12 and the blade 11. The skin dome 23 is a small amount of piled up skin 21 in front of the optical blade 11. The height 53 and shape of the skin dome 23 depends on multiple factors, such as the shaving speed, shaving direction, skin smoothness, skin flexibility, use of shaving lubricants, pressure exerted on the skin by the hair cutting device, etc. An important function of the skin stretcher 12 is to reduce the variation in skin dome 23 dimensions. The more constant the skin dome 23 shape and dimensions are, the closure to the skin surface 21 the hair cutting laser 13 can be focused without causing too much skin irritation.

According to the invention, the slit dimensions can be adapted for controlling the skin dome 23 shape and height 53. Two important slit dimensions that may be adapted are the slit size 51 and the exposure 52. The slit size 51 is the width of the slit as defined by the distance between a back surface of the skin stretcher 12 and a front surface of the optical blade 11. Exposure 52 is the distance between the stretcher surface of the skin stretcher 12 and the blade surface of the optical blade 11, measured in a direction perpendicular to those surfaces (and the skin surface 21). Skin dome height 53 may, e.g., be increased by increasing the slit size 51 and/or decreasing the exposure 52 or decreased by decreasing the slit size 51 and/or increasing the exposure 52. Changing the slit size 51 and/or exposure 52 may be realized by moving the skin stretcher 12 and/or the optical blade 11 relative to the hair cutting device 10. The slit adapting means may comprise actuators for providing the required movements which are preferably also controlled by the processor 15. In practice, it may be easier to only move the skin stretcher 12 and not the optical blade 11. The optical blade 11 already comprises a lot of mechanical and electronic parts for enabling hair detection and cutting of the hairs 22. Adding mechanical actuators for moving the optical blade 11 may be more complex from a constructional point of view than providing actuators for moving the skin stretcher 12. Suitable parameter ranges for the slit size may, e.g., be about 0.5 and 2.0 mm. The exposure may, e.g., be chosen in the range 0 - 250 µm (micrometer).

It is to be noted that the skin stretcher 12 and/or the cutter unit 11 may have some additional freedom of movement for following irregularities of the skin or body parts to be shaved. For example, the skin stretcher 12 and/or the cutter unit 11 may be mounted to the hair cutting device 10 with some springs allowing the skin stretcher 12 and/or cutter unit 11 to follow the contours of the body part during shaving. The skin stretcher 12 and the cutter unit 11 may move in union relative to the hair cutting device 10 in order to follow the skin contours. It is, however, important that this additional freedom of movement is not so large that it interferes with the skin dome control process.

In an embodiment of the hair cutting device according to the invention, the hair cutting device 10 may comprise control means for manually setting the appropriate slit dimensions. The user may, e.g., be enabled to select a slit size 51 and/or an exposure 52 from a discrete or continuous range of available settings. For this purpose, control means 42 may be provided at the outer surface of the hair cutting device 10. The control means 42 are coupled to the processor 15 and may also enable controlling other functions of the hair cutting device 10. The control means 42 may, e.g., comprise as an on/off-switch for turning the device 10 on or off. Alternatively, the device may offer different settings for short hair and for longer hair. Each setting then corresponds to a different predetermined slit size and/or exposure. Also, different settings for shaving a beard, a head, arms and/or legs may be provided. The user may be allowed to put the skin stretcher 12 or the optical blade 11 in one of the predetermined settings manually or the selected settings may be received by the processor 15 which then controls one or more actuators for moving the appropriate parts of the device 10. Alternatively, the appliance could offer a "test shave" in which the detection sensors are enabled and the cutting laser beam 13 is temporarily disabled. The information is then used to either set or propose an optimal setting.

In preferred embodiments, the processor 15 is arranged to control the skin dome height 53 by adapting slit dimensions in response to measurements of parameters that are indicative of or have an influence on the skin dome height 53. For example, the light based hair detector may be adapted to detect the skin dome height 53. The processor 15 may then be arranged to control the actuators in dependence of the detected skin dome height 53. The light based hair detector is already equipped to distinguish hairs 22 from skin 21. The processor can thus easily be programmed to be able to detect the skin dome height 53. For example, the light based hair detector may be used to determine, at one or more selected heights above the skin-contact surface, whether there is skin tissue 21 present or not. When the skin dome height 53 is known, for example from the detection statistics, it can also be controlled by adapting the slit dimensions accordingly.

Optionally, the skin stretcher 21 comprises a pressure sensor for determining a pressure exerted on the stretcher surface and the processor 15 is further arranged to control the slit adapting means in dependence of the determined pressure. When a user pushes the hair cutting device 10 into the skin 21 with too much force, the chances of the focal point of the hair cutting laser 13 falling within the skin 21 is considerably increased. To avoid irritation of the skin 21, the slit dimension may then be adapted in order to reduce skin dome height 23 and thus irritation.

Additionally or alternatively, the skin stretcher 12 comprises a friction sensor for determining a friction between the stretcher surface and the skin surface 21 and the processor 15 is further arranged to control the slit adapting means in dependence of the determined friction. Friction of the skin surface 21 may depend on, e.g., skin type, use of shaving lubricants, hair length and pressure applied by the hair cutting device 10. More friction leads to a higher skin dome 23, which can be compensated by appropriate changes to the slit dimensions.

Optionally, also a means for determining a speed of the hair treatment device 10 relative to the skin surface 21 is provided and the processor 15 is further arranged to control the slit adapting means in dependence of the determined speed. For example, one or more accelerometers 41 may be used for determining the speed or changes in speed of the hair cutting device 10. The accelerometers 41 are coupled to the processor 15 and may be positioned in any part of the hair cutting device 10.

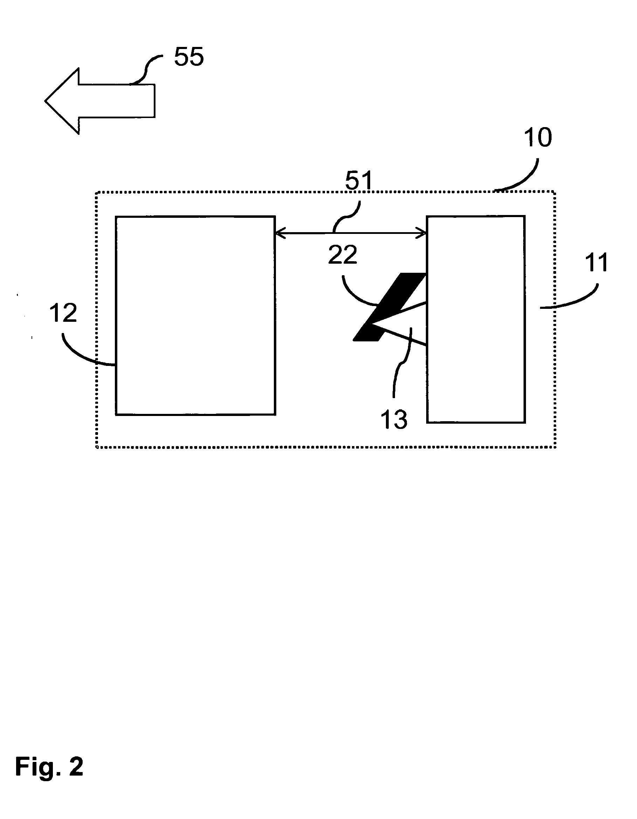

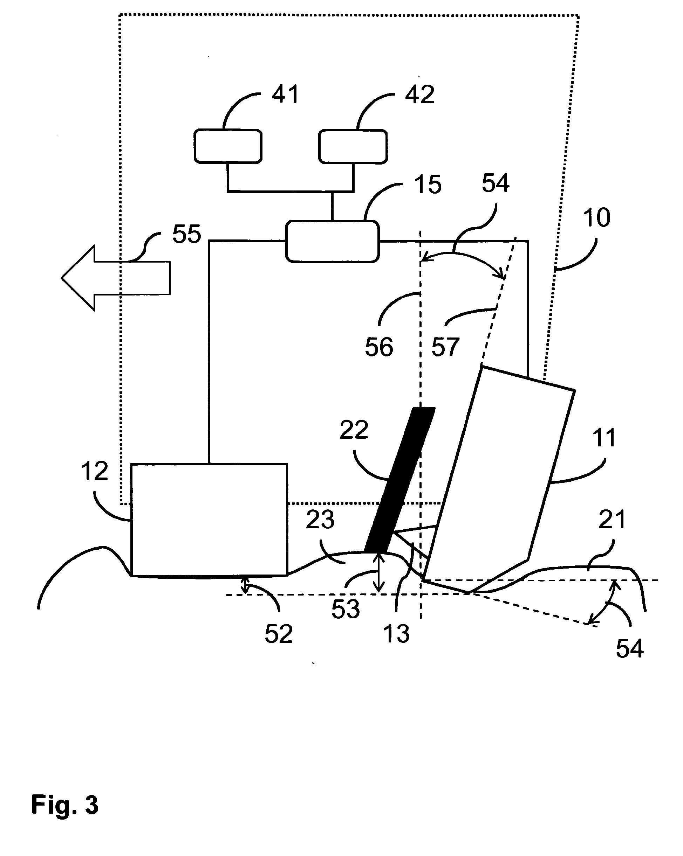

Figure 2 schematically shows a top view cross section of the hair cutting device 10 of figure 1. Figure 3 shows the cross section of figure 1, with the cutter unit 11 in a tilted position. In addition to adapting the slit size 51 and the exposure 52, also the cutter angle 54 between a neutral position 56 and a current position 57 of the front surface of the cutter unit 11 may be adapted. This cutter angle 54 is the same as the angle 54 between the skin-contact surface and the shaving direction 55. Changing the cutter angle 54 has two effects on the shaving process. A first effect is that the inclined skin-contact surface exerts a different pressure on the skin surface 21 than a flat skin-contact surface would do which may lead to a different dome shape and dome height 53. A second effect is that, because the light source is rotated together with the cutter unit 11, the focus of the hair cutting laser 13 will move away from or towards the skin surface 21. Both the change of the dome shape and the displacement of the focus 13 affect the closeness and the irritation. For optimum skin dome control, the rotation angle of the cutter unit is preferably selected somewhere in the range - 15° - 15°.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A hair cutting device (10) comprising:
- a cutter unit (11) comprising a skin-contact surface for dragging over a skin surface (21) in a shaving direction (55), a front surface arranged in front of the skin-contact surface in the shaving direction (55), and a laser beam exit window arranged in the front surface for allowing a hair cutting laser beam (13) to cut a hair (22) near the skin surface (21) in front of the front surface,
- a skin stretcher (12) being positioned in front of the cutter unit (11), according to the shaving direction (55), and comprising a stretcher surface for dragging over the skin surface (21) together with the skin-contact surface, such that a skin dome (23) is formed by the skin surface (21) in a slit between the skin stretcher (12) and the cutter unit (11), and
**characterized in that** the hair cutting device comprises a
- slit adapting means for adapting at least one dimension (51, 52, 54) of the slit for controlling a shape of the skin dome (23).

2. A hair cutting device (10) as claimed in claim 1, wherein the slit adapting means comprise means for adapting a slit size, the slit size being a distance between a rear surface of the skin stretcher (12) and the front surface of the cutter unit (11), measured in the shaving direction (55).

3. A hair cutting device (10) as claimed in claim 1, wherein the slit adapting means comprise means for adapting an exposure (52), the exposure (52) being a distance between the skin-contact surface and the stretcher surface measured in a direction perpendicular to the shaving direction (55).

4. A hair cutting device (10) as claimed in claim 1, wherein the slit adapting means comprise means for adapting an angle (54) between the skin-contact surface and the shaving direction (55).

5. A hair cutting device (10) as claimed in claim 1, wherein the slit adapting means comprise at least one actuator for moving the cutter unit (11) and/or the stretching element (12) relative to a housing of the hair cutting device (10).

6. A hair cutting device (10) according to claim 1, further comprising:
- a light based hair detector for detecting the hair (22) near the skin surface (21),
- a hair cutting laser source for generating the hair cutting laser beam (13), and
- a processor (15) coupled to the light based hair detector and
to the hair cutting laser source and being arranged to activate the hair cutting laser source in a focal position of the hair cutting laser beam (13) in which the light based hair detector has detected the presence of the hair (22).

7. A hair cutting device (10) as claimed in claim 6, wherein the processor (15) is further coupled to the slit adapting means for controlling the slit adapting means.

8. A hair cutting device (10) as claimed in claim 7, wherein the light based hair detector is further adapted to detect a height (53) of the skin dome (23) and wherein the processor (15) is further arranged to control the slit adapting means in dependence of the detected height (53) of the skin dome (23).

9. A hair cutting device (10) as claimed in claim 8, wherein the light based hair detector (10) employs a hair detecting laser beam for detecting the hair (22) and/or the skin surface (21) and wherein the height of the skin dome (23) is detected by determining whether the skin dome (23) is present at at least one selected height of the hair detecting laser beam in the slit.

10. A hair cutting device (10) as claimed in claim 7, wherein the skin stretcher (12) comprises a pressure sensor for determining a pressure exerted on the stretcher surface and wherein the processor (15) is further arranged to control the slit adapting means in dependence of the determined pressure.

11. A hair cutting device (10) as claimed in claim 7, wherein the skin stretcher (12) comprises a friction sensor for determining a friction between the stretcher surface and the skin surface and wherein the processor (15) is further arranged to control the slit adapting means in dependence of the determined friction.

12. A hair cutting device (10) as claimed in claim 7, further comprising means (41) for determining a speed of the hair treatment device relative to the skin surface (21) and wherein the processor (15) is further arranged to control the slit adapting means in dependence of the determined speed.

13. A hair cutting device (10) as claimed in claim 1, wherein the cutter unit comprises an optical blade (11) with a transparent blade body including the front surface and the laser beam exit window, wherein the blade body is adapted for guiding the hair cutting laser beam (13) to the exit window.

## Patentansprüche

1. Haarschneidevorrichtung (10), umfassend:
- eine Schneideinheit (11), umfassend eine Hautkontaktoberfläche zum Ziehen über eine Hautoberfläche (21) in eine Rasierrichtung (55), eine Vorderfläche, die vor der Hautkontaktoberfläche in Rasierrichtung (55) angeordnet ist, und ein Laserstrahl-Austrittsfenster, das in der Vorderfläche angeordnet ist, um es einem Haarschneide-Laserstrahl (13) zu ermöglichen, ein Haar (22) nahe der Hautoberfläche (21) vor der Vorderfläche zu schneiden,
- einen Hautspanner (12), der vor der Schneideinheit (11) gemäß der Rasierrichtung (55) angeordnet ist, und der eine Spannerfläche zum Ziehen über die Hautoberfläche (21) zusammen mit der Hautkontaktoberfläche umfasst, so dass eine Hautkuppe (23) von der Hautoberfläche (21) in einem Spalt zwischen dem Hautspanner (12) und der Schneideinheit (11) gebildet wird, und
**dadurch gekennzeichnet, dass** die Haarschneidevorrichtung Folgendes umfasst:
- ein Spaltanpassungsmittel zum Anpassen wenigstens einer Dimension (51, 52, 54) des Spalts, um eine Form der Hautkuppe (23) zu steuern.

2. Haarschneidevorrichtung (10) nach Anspruch 1, wobei das Spaltanpassungsmittel Mittel zum Anpassen einer Spaltgröße umfasst, wobei die Spaltgröße ein Abstand zwischen einer rückwärtigen Fläche des Hautspanners (12) und der Vorderfläche der Schneideinheit (11) ist, gemessen in Rasierrichtung (55).

3. Haarschneidevorrichtung (10) nach Anspruch 1, wobei das Spaltanpassungsmittel Mittel zum Anpassen einer Exposition (52) umfasst, wobei die Exposition (52) ein Abstand zwischen der Hautkontaktoberfläche und der Spannerfläche ist, gemessen in einer Richtung, die senkrecht zur Rasierrichtung (55) verläuft.

4. Haarschneidevorrichtung (10) nach Anspruch 1, wobei das Spaltanpassungsmittel Mittel zum Anpassen eines Winkels (54) zwischen der Hautkontaktoberfläche und der Rasierrichtung (55) umfasst.

5. Haarschneidevorrichtung (10) nach Anspruch 1, wobei das Spaltanpassungsmittel wenigstens ein Betätigungselement zum Bewegen der Schneideinheit (11) und/oder des Spannelements (12) in Bezug auf ein Gehäuse der Haarschneidevorrichtung (10) umfasst.

6. Haarschneidevorrichtung (10) nach Anspruch 1, ferner umfassend:
- einen lichtbasierten Haardetektor zum Erkennen des Haars (22) nahe der Hautoberfläche (21),
- eine Haarschneide-Laserquelle zum Erzeugen eines Haarschneide-Laserstrahls (13), und
- einen Prozessor (15), der an den lichtbasierten Haardetektor und an die Haarschneide-Laserquelle angeschlossen ist und der angeordnet ist, um die Haarschneide-Laserquelle in einer Fokusposition des Haarschneide-Laserstrahls (13) zu aktivieren, in der der lichtbasierte Haardetektor die Anwesenheit des Haars (22) erkannt hat.

7. Haarschneidevorrichtung (10) nach Anspruch 6, wobei der Prozessor (15) ferner an das Spaltanpassungsmittel angeschlossen ist, um das Spaltanpassungsmittel zu steuern.

8. Haarschneidevorrichtung (10) nach Anspruch 7, wobei der lichtbasierte Haardetektor ferner angepasst ist, um eine Höhe (53) der Hautkuppe (23) zu erkennen, und wobei der Prozessor (15) ferner angepasst ist, um das Spaltanpassungsmittel in Abhängigkeit von der erkannten Höhe (53) der Hautkuppe (23) zu steuern.

9. Haarschneidevorrichtung (10) nach Anspruch 8, wobei der lichtbasierte Haardetektor (10) einen Haarerkennungs-Laserstrahl zum Erkennen des Haars (22) und/oder der Hautoberfläche (21) einsetzt, und wobei die Höhe der Hautkuppe (23) erkannt wird durch Bestimmen, ob die Hautkuppe (23) bei wenigstens einer ausgewählten Höhe des Haarerkennungs-Laserstrahls im Spalt vorhanden ist.

10. Haarschneidevorrichtung (10) nach Anspruch 7, wobei der Hautspanner (12) einen Drucksensor zum Bestimmen eines Drucks umfasst, der auf die Spannerfläche ausgeübt wird, und wobei der Prozessor (15) ferner angeordnet ist, um das Spaltanpassungsmittel in Abhängigkeit von dem bestimmten Druck zu steuern.

11. Haarschneidevorrichtung (10) nach Anspruch 7, wobei der Hautspanner (12) einen Reibungssensor zum Bestimmen einer Reibung zwischen der Spannerfläche und der Hautoberfläche umfasst, und wobei der Prozessor (15) ferner angeordnet ist, um das Spaltanpassungsmittel in Abhängigkeit von der bestimmten Reibung zu steuern.

12. Haarschneidevorrichtung (10) nach Anspruch 7, ferner umfassend Mittel (41) zum Bestimmen einer Geschwindigkeit der Haarbehandlungsvorrichtung in Bezug auf die Hautoberfläche (21), und wobei der Prozessor (15) ferner angeordnet ist, um das Spaltanpassungsmittel in Abhängigkeit von der bestimmten Geschwindigkeit zu steuern.

13. Haarschneidevorrichtung (10) nach Anspruch 1, wobei die Schneideinheit eine optische Klinge (11) mit einem transparenten Klingenkörper umfasst, der die Vorderfläche und das Laserstrahl-Austrittsfenster umfasst, wobei der Klingenkörper angepasst ist, um den Haarschneide-Laserstrahl (13) zum Austrittsfenster zu lenken.

## Revendications

1. Dispositif de coupe de poil (10), comprenant :
- une unité de coupe (11) comprenant une surface entrant en contact avec la peau pour glisser sur une surface de peau (21) dans une direction de rasage (55), une surface avant agencée devant la surface, entrant en contact avec la peau dans la direction de rasage (55), et une fenêtre de sortie de faisceau laser, agencée dans la surface avant, pour permettre à un faisceau laser de coupe de poil (13) de couper un poil (22) près de la surface de peau (21) devant la surface avant,
- un étireur de peau (12) positionné devant l'unité de coupe (11), selon la direction de rasage (55), et comprenant une surface d'étireur pour glisser sur la surface de peau (21) conjointement avec la surface entrant en contact avec la peau, de sorte qu'un pli de peau (23) soit formé par la surface de peau (21) dans une fente entre l'étireur de peau (12) et l'unité de coupe (11), et
**caractérisé en ce que** le dispositif de coupe de poil comprend :
- des moyens d'adaptation de fente pour adapter au moins une dimension (51, 52, 54) de la fente pour commander une forme du pli de peau (23).

2. Dispositif de coupe de poil (10) selon la revendication 1, dans lequel les moyens d'adaptation de fente comprennent des moyens pour adapter une taille de fente, la taille de fente étant une distance entre une surface arrière de l'étireur de peau (12) et la surface avant de l'unité de coupe (11), mesurée dans la direction de rasage (55).

3. Dispositif de coupe de poil (10) selon la revendication 1, dans lequel les moyens d'adaptation de fente comprennent des moyens pour adapter une exposition (52), l'exposition (52) étant une distance entre la surface entrant en contact avec la peau et la surface d'étireur, mesurée dans une direction perpendiculaire à la direction de rasage (55).

4. Dispositif de coupe de poil (10) selon la revendication 1, dans lequel les moyens d'adaptation de fente comprennent des moyens pour adapter un angle (54) entre la surface entrant en contact avec la peau et la direction de rasage (55).

5. Dispositif de coupe de poil (10) selon la revendication 1, dans lequel les moyens d'adaptation de fente comprennent au moins un actionneur pour déplacer l'unité de coupe (11) et/ou l'élément étireur (12) par rapport à un logement du dispositif de coupe de poil (10).

6. Dispositif de coupe de poil (10) selon la revendication 1, comprenant en outre :
- un détecteur de poil à base de lumière pour détecter le poil (22) près de la surface de peau (21),
- une source laser de coupe de poil pour générer le faisceau laser de coupe de poil (13), et
- un processeur (15) couplé au détecteur de poil à base de lumière et à la source laser de coupe de poil et agencé pour activer la source laser de coupe de poil dans une position focale du faisceau laser de coupe de poil (13) dans laquelle le détecteur de poil à base de lumière a détecté la présence du poil (22).

7. Dispositif de coupe de poil (10) selon la revendication 6, dans lequel le processeur (15) est en outre couplé aux moyens d'adaptation de fente pour commander les moyens d'adaptation de fente.

8. Dispositif de coupe de poil (10) selon la revendication 7, dans lequel le détecteur de poil à base de lumière est en outre adapté pour détecter une hauteur (53) du pli de peau (23) et dans lequel le processeur (15) est en outre agencé pour commander les moyens d'adaptation de fente en dépendance de la hauteur détectée (53) du pli de peau (23).

9. Dispositif de coupe de poil (10) selon la revendication 8, dans lequel le détecteur de poil à base de lumière (10) utilise un faisceau laser détecteur de poil pour détecter le poil (22) et/ou la surface de peau (21) et dans lequel la hauteur du pli de peau (23) est détectée en déterminant si le pli de peau (23) est présent à au moins une hauteur sélectionnée du faisceau laser détecteur de poil dans la fente.

10. Dispositif de coupe de poil (10) selon la revendication 7, dans lequel l'étireur de peau (12) comprend un capteur de pression pour déterminer une pression exercée sur la surface d'étireur et dans lequel le processeur (15) est en outre agencé pour commander les moyens d'adaptation de fente en dépendance de la pression déterminée.

11. Dispositif de coupe de poil (10) selon la revendication 7, dans lequel l'étireur de peau (12) comprend un capteur de frottement pour déterminer un frottement entre la surface d'étireur et la surface de peau et dans lequel le processeur (15) est en outre agencé pour commander les moyens d'adaptation de fente en dépendance du frottement déterminé.

12. Dispositif de coupe de poil (10) selon la revendication 7, comprenant en outre des moyens (41) pour déterminer une vitesse du dispositif de traitement de poil par rapport à la surface de peau (21) et dans lequel le processeur (15) est en outre agencé pour commander les moyens d'adaptation de fente en dépendance de la vitesse déterminée.

13. Dispositif de coupe de poil (10) selon la revendication 1, dans lequel l'unité de coupe comprend une lame optique (11) avec un corps de lame transparent incluant la surface avant et la fenêtre de sortie de faisceau laser, dans lequel le corps de lame est adapté pour guider le faisceau laser de coupe de poil (13) vers la fenêtre de sortie.
